(19) Europäisches Patentamt
European Patent Office
Office européen des brevets

(11) **EP 2 208 463 B1**

(12) **EUROPEAN PATENT SPECIFICATION**

(45) Date of publication and mention
of the grant of the patent:
**12.03.2014 Bulletin 2014/11**

(51) Int Cl.:
*A61B 5/06* (2006.01)     *A61B 1/313* (2006.01)
*G06F 3/01* (2006.01)     *G06F 19/00* (2011.01)

(21) Application number: **10150423.1**

(22) Date of filing: **11.01.2010**

(54) **Interface between a surgeon and an automated assistant and method thereof**

Schnittstelle zwischen einem Chirurgen und einem automatischen Assistenten und entsprechendes Verfahren

Interface entre un chirurgien et un assistant automatisé et procédé correspondant

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **15.01.2009 US 144760 P**

(43) Date of publication of application:
**21.07.2010 Bulletin 2010/29**

(73) Proprietor: **M.S.T. Medical Surgery Technologies
LTD
17000 Upper Nazareth (IL)**

(72) Inventor: **Sholev, Mordehai
37830, Amikam (IL)**

(74) Representative: **Lecomte, Didier
Lecomte & Partners Sàrl
P.O. Box 1623
1016 Luxembourg (LU)**

(56) References cited:
**WO-A1-2009/010980     US-A- 5 443 066
US-A1- 2003 073 935**

• **ARSHAK K ET AL: "A Model for Estimating the
Real-Time Positions of a moving Object in
Wireless Telemetry Applications using RF
Sensors" PROCEEDINGS OF THE 2007 IEEE
SENSORS APPLICATIONS SYMPOSIUM, IEEE -
PISCATAWAY, NJ, USA, 1 February 2007
(2007-02-01), pages 1-6, XP031180429 ISBN:
978-1-4244-0677-7**

**Description**

TECHNICAL FIELD

This patent relates generally to endoscope guided surgical instruments and procedures, and in particular to interfaces that allow identification of the spatial position of a laparoscope during endoscopic surgery.

BACKGROUND ART

During laparoscopic surgery it is often required to shift the spatial placement of the endoscope in order to present the surgeon with an optimal view. Conventional laparoscopic surgery makes use of either human assistants who manually shift the instrumentation or alternatively of robotic automated assistants. Automated assistants utilize interfaces that enable the surgeon to direct the mechanical movement of the assistant, achieving a shift in the camera view. US patent 6,714,841 discloses an automated camera endoscope in which the surgeon is fitted with a head mounted light source that transmits his head movements to a sensor, forming an interface that converts said movements to directions for the mechanical movement of the automated assistant. Alternative automated assistants incorporate a voice operated interface, a directional key interface, or other navigational interfaces. The main disadvantage of the above interfaces is that they are based on cumbersome operations for starting and stopping movement directions that requires the surgeon's constant attention.

An article titled "A method for estimating the real time Positions of a moving object in wireless telemetry applications using RF sensors" by K. Arshak was recently published (IEEE Sensors Applications Symposium, San Diego, California, USA, 6-8 February 2007). The article relate to locating a transmitting object using **multiple receiving antenna sensors** located at various place surrounding the transmitting device. The receiver antennas are assumed to be omni directional and the location of the transmitter is achieved through distance estimation (i.e., triangulation) from each of the receiving antenna.

The distance from the transmitter is estimated by measuring the received signal strength (RSS) of the received signal:

$$RSS = P_T - P_L(d_0) - 10\eta \, \log_{10}(d/d0) + X_\sigma,$$

calculated in decibel, $P_T$ is the transmitted power, $P_L(d_0)$ is the path loss for a reference distance do, $\eta$ is the pass loss exponent, d is the distance between the transmitter and the receiver and $X_\sigma$ is a Gaussian random variable.

Therefore, the signal received is proportional to transmit power ($P_T$), the $\eta$ power of distance to the transmitter, normally in free space $\eta = 2$, if all is known except the distance, it can be resolved from the above mentioned equation, a distance can be calculated from each of the receiving antenna

The object (transmitter) location is at the intersection of spheres with every distance which is the respective sphere radius (i.e., Triangulation).

**Arshak** states in the article that other method such as time of arrival, time differences of arrival and angle of arrival are **not feasible** due to dense multipath environment. Yet more, if the transmit power is unknown, unstable or inaccurate; or if the propagation factor is unknown then **Arshak's** method won't be valid.

Therefore there is still a long felt need for a method which will enable the relative position of the transmitter (and thus the medical instrument).

US application no. US2003073935 to OLYMPUS OPTICAL CO relates to a capsulated body having a facility, which acquires biomedical information through endoscopic examination or the like, incorporated therein is inserted into a duct within a living body. Whether the capsulated body has halted for a certain period of time is detected from the time-varying position of the capsulated body or a time-passing change in an image. If it is judged that the capsulated body has halted at a stenosed region or the like, the fact is notified so that the capsulated body can be collected immediately.

US patent no. US5443066 to GEN ELECTRIC relates to an invasive imaging system employs a self-contained RF transmitter attached to an invasive device which allows tracking of the invasive device within a subject without physical connections to a tracking/display system and without the use of ionizing rays. An imaging system obtains a medical diagnostic image of the subject. The self-contained RF transmitter is comprised of a power generator means, a power conversion means such as an oscillator which converts the generated power to a radiofrequency (RF) signal, and a broadcasting means such as a tuned transmit coil for radiating the RF signal. The radiated RF signal is received by receive coils of a tracing/display means which calculates the location of the RF transmitter. The tracking/display means displays the medical diagnostic image on a monitor and superimposes a symbol on the image at a position corresponding to the calculated location of the RF transmitter. Prior to operation, the power generator may be energized inductively, photo-voltaically, or by direct contact with an external power supply. The power generator means may also be a charged battery sealed inside the RF transmitter. The RF transmitter may be implanted to track the motion of internal tissues.

However, both publications calculates the position of the object by means of triangulation. **As stated above,** if the transmit power is unknown, unstable or inaccurate; or if the propagation factor is unknown then **Triangulations** method (e.g. US5443066 or US2003073935) won't be valid.

Therefore there is still a long felt need for a method which will enable the relative position of the transmitter (and thus the medical instrument).

Research has suggested that these systems divert the surgeons focus from the major task at hand. Therefore technologies based on various kinds of positioning systems have been developed to simplify interfacing control. These technologies still fail to address another complicating interface aspect of laparoscopic surgery, however, as they do not allow the surgeon to signal both to the automated assistant and to surgical colleagues on which surgical instrument his attention is focused.

Thus, there is a long-felt need for a device that would allow the surgeon to identify to the laparoscopic computing system as well as to surgical colleagues to which surgical instrument attention is to be directed, thereby directing the view provided by the endoscope to the selected area of interest.

SUMMARY OF THE INVENTION

The present invention is defmed by the scope of independent claims 1 and 11.

In accordance with a preferred embodiment of the present invention, it is an object of the present invention to disclose an interface between a surgeon and an automated assistant, comprising (a) at least one array comprising $N$ RF transmitters, where $N$ is a positive integer; (b) one RF receiver, provided with at least two directional antenna; (c) means for attaching said RF transmitter array to at least one surgical tool; and, (d) a computerized operating system adapted to record the received signal strength (RSS) received by said RF receiver and to calculate therefrom the position of each of said N RF transmitters, and further adapted to provide automatically the results of said calculation to the human operator of said interface. It is within the essence of the invention wherein said computerized operating system calculates at least one of the parameters chosen from the group consisting of (a) the angle from which the signal had been received; (b) the spatial location of said at least one surgical tool; (c) the path of said at least one surgical tool; (d) the spatial location of the point of insertion of said at least one surgical tool into the body of a patient; (e) the spatial location of the tip of said at least one surgical tool; (f) matching each RF transmitter code with each calculated spatial location of said at least one surgical tool and/or said tip of said at least one surgical tool, and further wherein said computerized operating system provides automatically the results of said calculation to the human operator of said interface.

It is a further object of this invention to disclose such an interface, further comprising an endoscopic device.

It is a further object of this invention to disclose such an interface, wherein said endoscopic device comprises optical imaging means, and further wherein said computerized operating system calculates at least one of the parameters chosen from the group consisting of (a) the spatial location of said at least one surgical tool; (b) the path of said at least one surgical tool; (c) the spatial location of the point of insertion of said at least one surgical tool into the body of a patient; (d) the spatial location of the tip of said at least one surgical tool; (e) matching each RF transmitter code with each calculated spatial location of said at least one surgical tool and/or said tip of said at least one surgical tool; (f) the predicted appearance of said at least one surgical tool within said optical image; (g) if more than one of said at least one surgical tools appears simultaneously in said optical image, distinguishing among said more than at least surgical tools appearing in said optical image, and further wherein said computerized operating system provides automatically the results of said calculation to the human operator of said interface.

It is a further object of this invention to disclose such an interface, further comprising (a) a automated assistant for said endoscopic device; and (b) means for interfacing said computerized operating system to said automated assistant. It is within the essence of the invention wherein said computerized operating system calculates at least one of the parameters chosen from the group consisting of (a) the spatial location of said at least one surgical tool; (b) the path of said at least one surgical tool; (c) the spatial location of the point of insertion of said at least one surgical tool into the body of a patient; (d) the spatial location of the tip of said at least one surgical tool; (e) matching each RF transmitter code with each calculated spatial location of said at least one surgical tool and/or said tip of said at least one surgical tool; (f) a desired new location for said endoscopic device; (g) command protocol means for directing said automated assistant via said interface to maneuver said endoscopic device to a desired new location, and further wherein said computerized operating system provides automatically the results of said calculation to the human operator of said interface.

It is a further object of this invention to disclose such an interface, wherein said endoscopic device comprises optical imaging means, and further wherein said computerized operating system calculates at least one of the parameters chosen from the group consisting of (a) the spatial location of said at least one surgical tool; (b) the path of said at least one surgical tool; (c) the spatial location of the point of insertion of said at least one surgical tool into the body of a patient; (d) the spatial location of the tip of said at least one surgical tool; (e) matching each RF transmitter code with each calculated spatial location of said at least one surgical tool and/or said tip of said at least one surgical tool; (f) the predicted appearance of said at least one surgical tool within said optical image; (g) if more than one of said at least one surgical tools appears simultaneously in said optical image, distinguishing among said more than at least surgical tools appearing in said optical image; (h) a desired new location for said optical imaging means; (i) a command protocol for directing said automated assistant via said interface to maneuver said endoscopic device to a desired new location, and further wherein said computerized operating system provides automatically the results of said calculation to the human operator of said interface.

It is a further object of this invention to disclose such an interface, wherein said computer controller additionally transmits a command protocol to said automated assistant via said interface to maneuver said endoscopic device to a desired new location.

It is a further object of this invention to disclose such an interface, wherein said interface is adapted for manual operation, whereby each of said N transmitters transmits in response to a command signal from the human operator of the interface.

It is a further object of this invention to disclose such an interface, wherein said interface is adapted for automatic operation, whereby each of said N transmitters transmits continuously.

It is a further object of this invention to disclose such an interface, wherein said interface is adapted for automatic operation, whereby each of said N transmitters transmits continuously and further wherein said computer transmits said calculated parameters for each of said $N$ transmitters in response to a command signal from the human operator of the interface.

It is a further object of this invention to disclose such an interface, wherein said antenna array comprises at least one directional antenna.

It is a further object of this invention to disclose such an interface, wherein said transmitters transmit in the 430 MHz ISM band.

It is a further object of this invention to disclose such an interface, wherein $M = 1$, and further wherein said receiver array is adapted to determine the angle whose vertex is the location of said antenna array and which is subtended by the line connecting any two of said $N$ transmitters.

It is a further object of this invention to disclose such an interface, wherein said interface comprises $M$ receivers, $M$ is an integer higher than 1; and further wherein said M receivers are adapted to determine the location of each of said $N$ transmitters by triangulation.

It is a further object of this invention to disclose such an interface, wherein said transmitters transmit a modulated signal, said modulation chosen from the group consisting of (a) frequency modulation, (b) amplitude modulation.

It is a further object of this invention to disclose such an interface, wherein said modulation occurs at a frequency of about 1.5 kHz.

It is a further object of this invention to disclose such an interface, wherein each of said $N$ RF transmitters is modulated at a different frequency.

It is a further object of this invention to disclose such an interface, wherein said $N$ modulation frequencies are chosen from the band of frequencies spanning the range of from about 1.0 kHz to about 1.5 kHz.

It is a further object of this invention to disclose such an interface, wherein receiver is a single conversion receiver.

It is a further object of this invention to disclose a method for calculating positional parameters of a laparoscopic surgical tool, comprising the steps of (a) obtaining an interface for a laparoscope, said interface comprising (*i*) at least one array comprising $N$ RF transmitters, where $N$ is a positive integer, (*ii*) one RF receiver provided with at least two directional antenna; (*iii*) a computerized operating system adapted to record the received signal strength RSS received by each antenna of said RF receiver and to calculate therefrom the position of each of said $N$ RF transmitters, and further adapted to provide automatically the results of said calculation to the human operator of said interface; (b) obtaining a surgical tool; (c) attaching said RF transmitter array to said surgical tool; (d) measuring the received signal strength (RSS) from said $N$ RF transmitters received at each of said directional antenna of said RF receivers; and (e) calculating spatial parameters relating to each of said $N$ transmitters according to a predetermined protocol. It is in the essence of the invention wherein said step of calculating said parameters of each of said $N$ transmitters yields positional parameters of said laparoscope surgical tool, said positional parameters is selected from a group consisting of (a) the angle from which the signal had been received; (b) the spatial location of said at least one surgical tool; (c) the path of said at least one surgical tool; (d) the spatial location of the point of insertion of said at least one surgical tool into the body of a patient; (e) the spatial location of the tip of said at least one surgical tool; (f) matching each RF transmitter code with each calculated spatial location of said at least one surgical tool and/or said tip of said at least one surgical tool, and further wherein said computerized operating system provides automatically the results of said calculation to the human operator of said interface.

Also disclosed, but not forming part of the claimed invention, is a method for controlling the position of an endoscopic device, comprising the steps of (a) obtaining an interface between a surgeon and an automated assistant, said interface comprising (*i*) at least one array comprising *N* RF transmitters, where N is a positive integer, (*ii*) one RF receiver provided with at least two directional antenna; (*iii*) a computerized operating system adapted to record the received signal strength RSS received by each antenna of said RF receiver and to calculate therefrom the position of each of said *N* RF transmitters, and further adapted to provide automatically the results of said calculation to the human operator of said interface; (*iv*) an automated assistant for said endoscopic device; and, (*v*) means for interfacing said computerized operating system to said automated assistant; (b) obtaining a surgical tool; (c) attaching said RF transmitter array to said surgical tool; (d) measuring the received signal strength (RSS) from said *N* RF transmitters received at each of said directional antenna of said RF receivers; (e) calculating spatial parameters relating to location of each of said *N* transmitters; (f) calculating a desired new position for said endoscopic device; (g) sending a command from said computerized operating system to said automated assistant via said interfacing means to maneuver said endoscopic device to said desired new location; and, (h) maneuvering said endoscopic device to said desired new location.

Said step of calculating said parameters of each of said *N* transmitters yields positional parameters of said laparoscope surgical tool, said positional parameters is selected from a group consisting of (a) the angle from which the signal had been received; (b) the spatial location of said at least one surgical tool; (c) the path of said at least one surgical tool; (d) the spatial location of the point of insertion of said at least one surgical tool into the body of a patient; (e) the spatial location of the tip of said at least one surgical tool; (f) matching each RF transmitter code with each calculated spatial location of said at least one surgical tool and/or said tip of said at least one surgical tool, and further wherein said computerized operating system provides automatically the results of said calculation to the human operator of said interface.

It is a further object of this invention to disclose such an interface, wherein said endoscopic device comprises optical imaging means, and further comprising the additional steps of (a) determining said position of said surgical tool relative to the image frame; and (b) maneuvering said optical imaging means such that said surgical tool appears at a predetermined location within said image frame.

It is a further object of this invention to provide such a method, wherein each of said *N* transmitters transmits in response to a signal from the human operator of said interface.

It is a further object of this invention to provide such a method, wherein each of said *N* transmitters transmits continuously.

The device of the present invention has many technological advantages, among them:

- Simplifying the communication interface between surgeon and automated assistants.
- Seamless interaction with conventional computerized automated endoscope systems.
- Simplicity of construction and reliability.
- User-friendliness.
- Additional features and advantages of the invention will become apparent from the following drawings and description.

BRIEF DESCRIPTION OF DRAWINGS

For a better understanding of the invention with regard to the embodiments thereof, reference is made to the accompanying drawings (not to scale), in which like numerals designate corresponding sections or elements throughout, and in which:

Fig. 1A shows a general schematic view of surgical tool positioning system that detects the location of a surgical tool at the time the surgeon activates the surgical tool transmitter (manual mode);

Fig. 1B shows a general schematic view of surgical tool positioning system that detects the location of surgical tools continuously (continuous automatic mode);

Fig. 2A is a diagram demonstrating sequential transmit operation;

Fig 2B is a diagram demonstrating periodic transmit operation, with unequal rates for left and right transmitters;

Fig 2C is a diagram demonstrating simultaneous transmit operation with different frequencies;

Figs. 3a and 3b is a schematic view of the antenna pattern;

Fig. 4A shows the system block diagram;

Fig. 4B shows antenna set block diagram;

Fig. 4C shows the receiver internal block diagram;

Fig. 4D shows the controller / sequencer block diagram;

Fig. 5 shows system control software operation flow;

Fig 6A shows the antenna switching pattern during periodic transmit operation;

Fig 6B shows the antenna switching pattern during sequential transmit operation;

Fig 7A shows an example of a planar antenna structure;

Fig 7B shows an example of a spatial antenna structure;

Fig 7C shows an example of a spatial antenna mounted on the camera holder mechanism;

Fig 8A shows an example of using the location system in abdominal laparoscopic surgery;

Fig 8B shows an example of using the location system in knee endoscopic surgery; and,

Fig 8C shows an example of using the location system in shoulder endoscopic surgery.

DETAIL DESCRIPTION OF THE INVENTION

The present invention is utilized to improve upon the interface between surgeon and automated assistants by communicating the surgeon's current instrument of choice, supplying location data to the image processing computing software thereby directing the endoscope to focus on said choice. The technology relies on marrying a conventional laparoscopic system with data obtained from small RF transmitters attached to a surgical tool. It will be apparent to one skilled in the art that there are several embodiments of the invention that differ in details of construction, without affecting the essential nature thereof, and therefore the invention is not limited by that which is illustrated in the figures and described in the specification, but only as indicated in the accompanying claims, with the proper scope determined only by the broadest interpretation of said claims.

The present invention provides an interface between a surgeon and an automated assistant, comprising (a) at least one array comprising *N* RF transmitters, where *N* is a positive integer; (b) one RF receiver, said receiver provided with at least one directional antenna; (c) means for attaching said RF transmitter array to at least one surgical tool; and, (d) a computerized operating system adapted to record the relative signal strength received by said RF receiver and to calculate therefrom the position of each of said *N* RF transmitters, and further adapted to provide automatically the results of said calculation to the human operator of said interface. It is within the essence of the invention wherein said computerized operating system calculates at least one of the parameters chosen from the group consisting of (a) the spatial location of said at least one surgical tool; (b) the path of said at least one surgical tool; (c) the spatial location of the point of insertion of said at least one surgical tool into the body of a patient; (d) the spatial location of the tip of said at least one surgical tool; (e) matching each RF transmitter code with each calculated spatial location of said at least one surgical tool and/or said tip of said at least one surgical tool, and further wherein said computerized operating system provides automatically the results of said calculation to the human operator of said interface.

As used herein, the term **'automated assistant'** refers to any mechanical device (including but not limited to a robotic device) that can maneuver and control the position of a surgical or endoscopic instrument, and that can in addition be adapted to receive commands from a remote source.

As used herein, the term **'Antenna Gain'** refers to the ratio of the <u>radiation</u> intensity of an <u>antenna</u> in a given direction to the intensity that would be produced by a hypothetical ideal antenna that radiates equally in all directions (isotropically) and has no losses.

As used herein, when referring to transmission of information to a human, the term **'provide'** refers to any process (visual, tactile, or auditory) by which an instrument, computer, controller, or any other mechanical or electronic device can report the results of a calculation or other operation to a human operator.

As used herein, the term **'automatic'** or **'automatically'** refers to any process that proceeds without the necessity of direct intervention or action on the part of a human being.

In one of the preferred embodiments of the invention, any desired surgical instrument is fitted with an RF transmitter, and selection is achieved by depressing its button.

The invention describes two methods of operation:

A **manual method,** in which a transmitter emits RF signal *only when* the surgeon presses a button located e.g., on one of the arms (either left or right - but not both simultaneously), the system then indicating the direction of that arm; and,

An **automatic method,** in which *all transmitters continuously emit* RF signal and the system tracks the direction of all transmitters simultaneously. When the surgeon presses a button of one of the transmitters, the system output is the direction and location of the specific transmitter.

The term **"about"** refers hereinafter to a range of 25% below or above the referred value.

The automatic mode has some advantages over the manual mode because the system can make use of history track files in order to filter the data and apply prediction algorithms. The continuous stream of data also allows the software to compute additional important data such as the insertion point of each tool, and the predicted tools location on the image.

System operation will be explained for both MANUAL-(sequential), and AUTOMATIC (periodic or simultaneous) modes. In order to simplify the explanation a system used to locate the positions and directions of only 2 surgical tools is described, but the method described can be used with minor changes to locate the position of any number of surgical tools used in any laparoscopic surgeries.

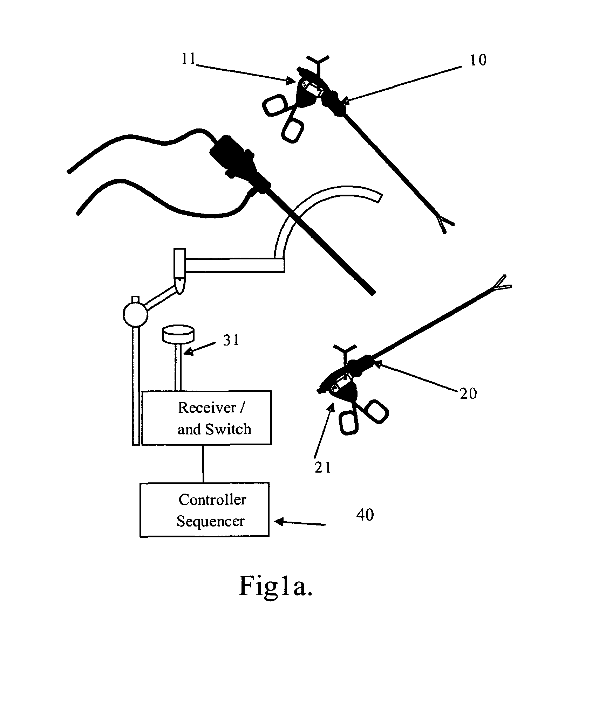

Reference is now made to **Fig 1a** which schematically describes the surgical tool positioning MANUAL system according to one embodiment of the invention. An antenna **31** is set and a receiver is preferably mounted on, or near, the robotic camera holder. Two identical transmitters, i.e., (i) transmitter **11** mounted on surgical tool **10;** and (ii), transmitter **21** mounted on surgical tool **20** are provided. A control and processing function controller **40** is further provided, being either a laptop PC or an embedded controller.

As described above, in the MANUAL system the transmitter emits RF signal *only when* the surgeon presses upon the surgical instrument the surgeon desires to track. Once the transmitter transmits a signal, the receiver communicates with the controller and instructs the tracking of the medical instrument desired by the surgeon.

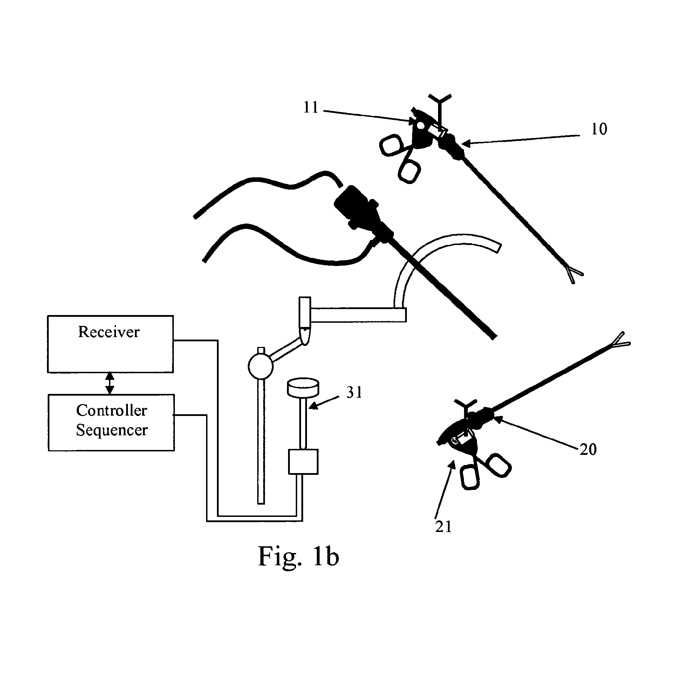

Reference is now made to **Fig 1b** which schematically describes the surgical tools positioning AUTOMATIC system according to yet another embodiment of the invention.

As described above, in the AUTOMATIC system the transmitter *continuously* emits RF signals. Therefore, the receiver constantly communicates with the controller.

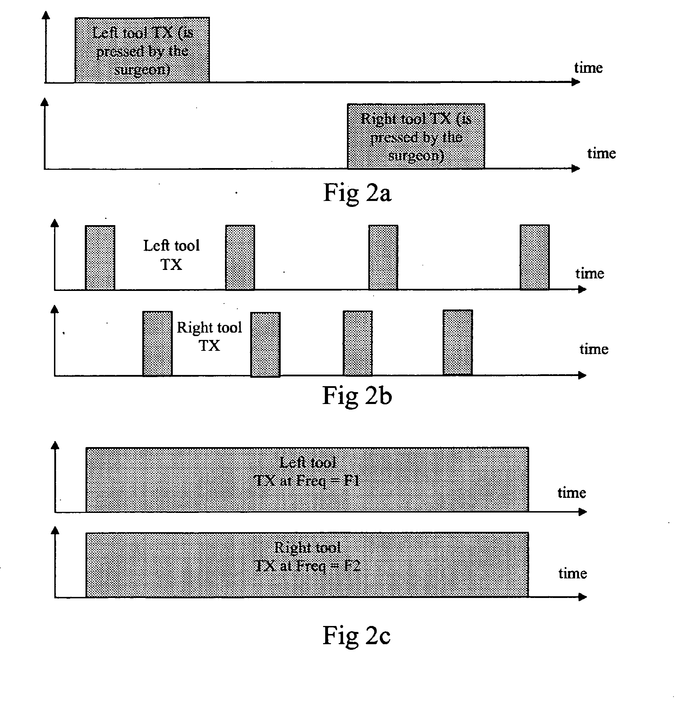

The transmitters **11** and **21** can operate in one of three modes: (a) sequential/manual mode, as shown in Figure 2a, upon the surgeon's pressing an appropriate button (manual mode); (b) periodic/automatic mode as shown in Figure 2b, in which the transmitters attached to the two tools provide pulsed signals at different pulse rates; or (c) simultaneous/ automatic mode, as shown in Figure 2c, in which the two transmitters transmit simultaneously and continuously, but at different radio frequencies. In all three modes, the receiver can detect and process individual reception from any one of the two tools and identify which transmission belongs to which tool.

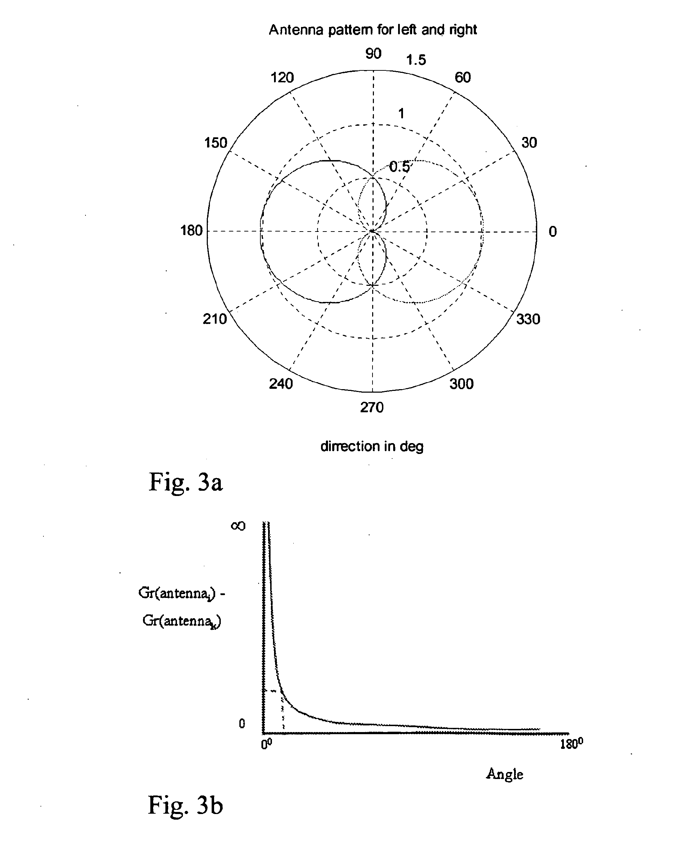

Reference is now made to Figures 3, 4a through 4d, and 5, in which further details of the system operation are illustrated.

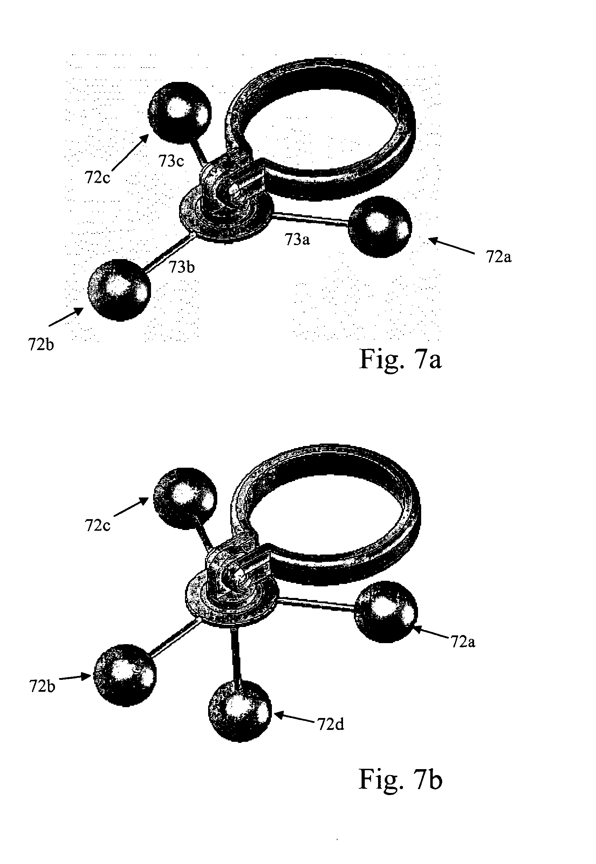

The receiver receives sequentially the signal of each tool through the antenna set, the antenna set comprising at least one (preferably) multiple directional antennas array as shown in Fig. 7a, where at least one of the antenna is connected to the receiver.

In order to locate the transmitter, at least two directional patterns are required as illustrated in Fig. 3a. The figure illustrates a typical antenna's pattern as a function of the signal's angle and of the intensity.

Alternatively, the transmitted signal may be modulated (in different embodiments of the invention, either (a) frequency modulation or (b) amplitude modulation or (c) both FM and AM simultaneously). Thus, in order to identify the arm from which the transmission is being received, each transmitter has a different modulation frequency. Hence an easier detection of the arm is enabled.

In general once the correct transmitter has been identified, the following mathematical analysis is performed:

The direction of each transmitter (and hence the desired instrument) is calculated by using a at least one receiver, preferably one receiver, having at least 2 directional antennas, preferably 3.

The received signal strength (RSS) is a function of the distance (d) between the receiver and the transmitter (i.e., the instrument); the strength of the transmitted signal (P); the path loss exponent ($\eta$); and the antenna's gain (Gr).

Since all the antenna are co-located, the ratio of the RSS will be the ratio between the antennas' gain.

Therefore, by knowing how the gain's ratio varies with the angle - one can calculate the angle from which the signal has been transmitted.

The above mentioned mathematical analysis is performed based on the following facts:

1. The method uses several directional antenna that are co-located as a set of receiving antenna; and,
2. The transmitter is assumed to be located somewhere around the receiving antenna set.

As described, the method is adapted to find **only** the direction of the transmit antenna by comparing the received power from all antenna in the set.

As commonly known, the received power depend on the transmit power ($P_T$), the distance from receiving to transmit antenna (d) and on the receiving antenna gain (Gr(i)) in the direction of the transmitter.

Since the set of antenna are co-located (the transmit power ($P_T$), the distance from receiving to transmit antenna (d) et cetera are eliminated) and the ratio of the receiving signal strength (RSS) is as follows:

$$\text{RSS(antenna}_i) / \text{RSS(antenna}_k) = \text{Gr(antenna}_i) / \text{Gr(antenna}_k)$$

As can be seen, the difference in the RSS does not depend upon the transmit power $P_T$ (since the $P_T$ received by each antenna is the same), and it does not depend upon the distance (since the received antenna are co-located).

Since the received antenna are directional, the gain pattern is unambiguously depend upon the angular positioning of the transmitter (and hence the instrument). Therefore, the angular position and hence the direction can be resolved in unambiguously manner from the gain difference.

Therefore, by knowing how the gain's ratio varies with the angle - one can calculate the angle from which the signal has been transmitted (see figure 3b). As illustrated in figure 3b, once the RSS differences is know, the angle is from which the signal is being sent (i.e., the angular location of the transmitter and hence the instrument) can be calculated.

It should be noted that the above mentioned calculation is much less sensitive to multipath environment found whilst applying the methods in laparoscopic surgeries.

According to another embodiment of the invention, the antenna array has more than two patterns, allowing the system to identify the direction of the tool with a finer resolution. Reference is now made to Fig. 7b, in which a non-limiting example of an additional embodiment is illustrated, in which the antenna array comprises four directional patterns: left, right, forward and aft. From the direction from which the strongest reception is received, the system is able to identify the sector in space in which the tool is located. Moreover, from interpolation of the received power from all antenna patterns, even finer directional resolution is possible.

The receiver detects the received signal power for each antenna in the array and reports it to the controller. The controller then resolves the directions of the two tools relative to antenna **31.**

Transmitters **11** and **21** shown in Fig. 1a may transmit in a wide range of frequencies; a typical frequency is the ISM band of 430MHz. Transmission is done at very low power, generally below about 1 mW.

The transmitted signal is modulated (in different embodiments of the invention, either (a) frequency modulation or (b) amplitude modulation or (c) both FM and AM simultaneously). In a preferred embodiment, the modulation is performed at an audio rate of about 1.5 kHz The transmitter uses a built-in antenna. In order to identify the arm from which the transmission is being received, each transmitter has a different modulation frequency. In a preferred embodiment of the invention, the frequencies are located within the band encompassing the range of from about 1.0 kHz to about 1.5 kHz.

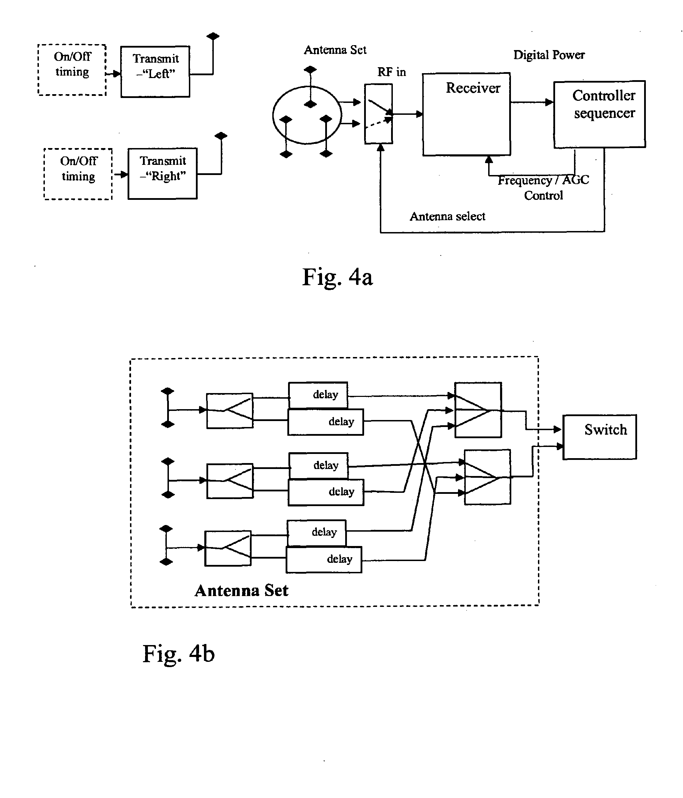

The signal for each transmitter is received by all antennas in the array (see Fig. 4b above). The antennas in the array typically comprise three very short dipoles mounted on the edge of an equal edge three-legged star or circle, as shown in figures 7a and 7b. The diameter of the circle or three-legged star is about 8 to 12 cm for operation at about 430 MHz. The use of the antenna array to identify the beam pattern is illustrated schematically in Fig. 4b. The antenna pattern is formed by combining the signal received by each antenna with different delays and signal weights. In order to set the pattern, in a typical embodiment, each antenna output is split into several equal power signals and a sample of each antenna signal is combined into one directional output. Which output is being measured is selected by an external switch.

The receiver receives the signal in sequence from each directional pattern and detects the signal power in any pattern for the signals from both tools; from the power ratio the signal direction is calculated. For example, for a two pattern antenna (left and right) if the signal from left antenna is much stronger than from right one, then the signal must have arrived from the left and vice versa. In parallel, the signal modulation as transmitted is detected and the modulation frequency is measured. Since each transmitter has a different modulation frequency, identification of the transmitter from which a particular signal originates is straightforward.

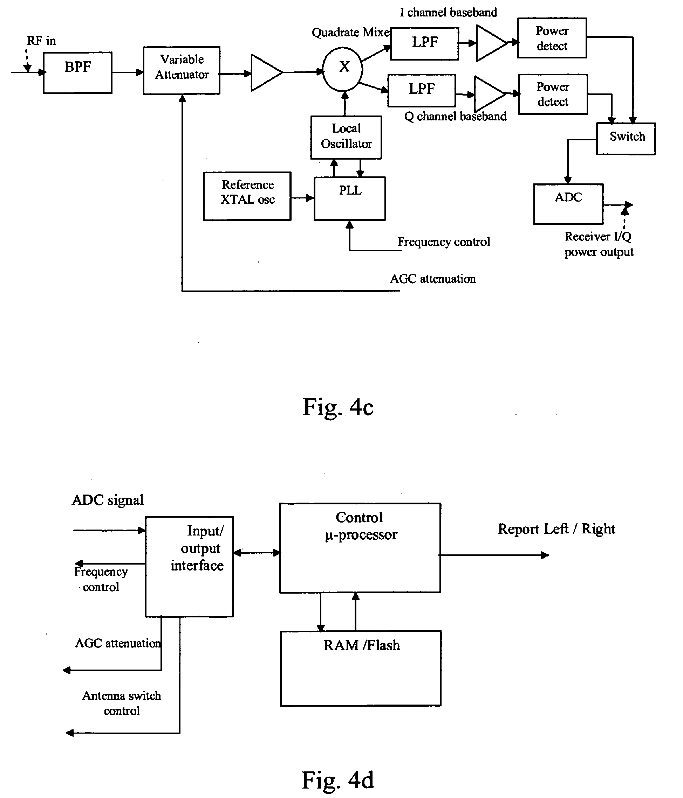

The receiver may be of any type, but in order to reduce the cost, size and power consumption, in a preferred embodiment, the receiver is a single conversion receiver that converts the input signal to base band. The receiver block diagram is shown in Fig. 4c. The receiver operation is as follows: the RF input is filtered around the transmitter frequency band then passed through a variable attenuator controlled by the system controller. Next, it is then amplified and then down converted using a Quadrate mixer and a local oscillator. The mixer outputs are the IF baseband: I (in phase) and Q (Quadrate) outputs, which are filtered by two low pass filters (e.g., about 30 kHz) then amplified. The base band signal powers are then detected. The DC power relative to the signal power is selected in sequence. The analog signal is then passed to an analog to digital converter (ADC), following which the total received power is computed digitally.

In an additional embodiment of the invention (not illustrated), the base band signal is analog to digital converted, so that the power of both the I and the Q channel is converted to a digital value.

The local oscillator frequency is locked by the PLL to the XTAL reference oscillator, controlled by the system controller.

In order to ensure that received signal is within a limited range the receiver gain is adjusted automatically (AGC).

Finally, as shown in Fig. 4d, the digital signal power is transferred to the system controller, where the controller calculates from the time of reception from which tool it is received and from which antenna pattern, using the power the controller compute the tool direction for each of the two tools.

The controller includes a timer based sequencer, preferably built into the microprocessor timing unit, that switches the receiver antenna, and in case of multiple frequency transmission, sets the receiver frequency sequentially.

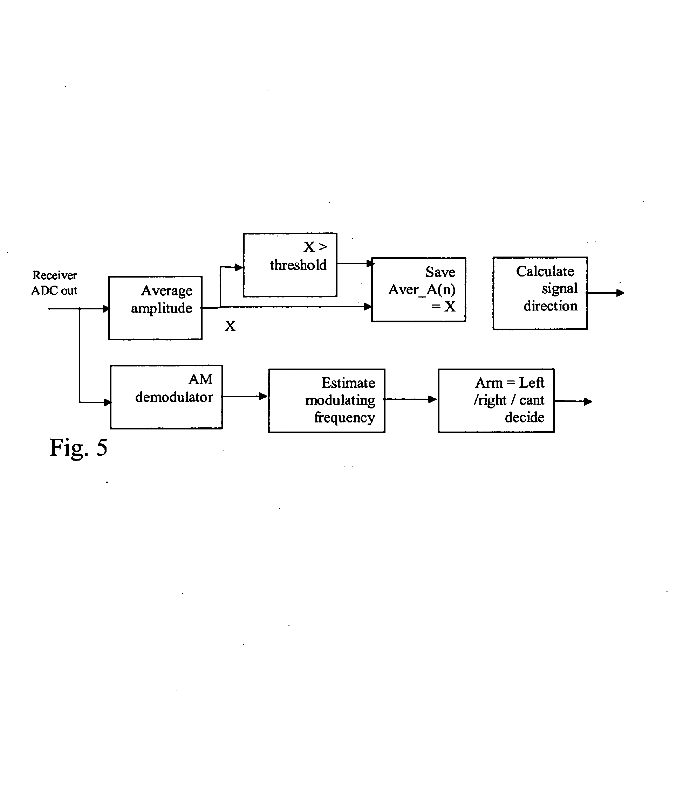

The operation sequence of the system is illustrated schematically in fig. 5, which shows the system control software operation flow:

[1] The AD signal is averaged to detect the average amplitude, averaging being done over one dwell duration ("X" indicates the output after averaging);

[2] Signal presence is detected when X is above a predetermined threshold;

[3] Average amplitude X is saved in a vector array (Aver_A (n), n ={1,2,.. N}) if signal is present, the storage being done on the appropriate antenna number place in the array;

[4] If for a given antenna, signal is present on N successive dwell durations, the signal direction is calculated;

[5] The modulation (in the particular embodiment illustrated, AM) is detected from the signal power input;

[6] The modulation frequency is measured; and,

[7] From the measured frequency, the arm type is detected; in the case of weak signal or simultaneous transmission, the module reports "can't decide," indicating a garbage signal.

In embodiments in which the transmitter operates periodically, both transmitters operate for a fraction of the time then switch off, then switch on again and so on with a constant or random cycle periodicity, each transmitter transmitting with a different transmission pulse cycle time in order to ensure that transmissions will not overlap at all times but only at times separated by $t_1 \cdot t_2$, where $t_1$ and $t_2$ are the pulse cycle times of the two transmitters. In parallel, the receiver sequentially switches the receiver channel among the different antennas and dwells on each antenna for a fixed dwell time. From the level of signal received, the system determines whether or not a signal is present. If a single signal is present either from the right arm or left arm transmitter, the direction of the signal is calculated from the signal strength received from different antennas, and the arm is identified from the internal modulation frequency. In case of coincident simultaneous transmit the receiver cannot identify the signal modulation therefore the measurement is rejected. In an additional embodiment, the system tracks the transmission period cycle of each arm and predicts the simultaneous transmission times in order better to identify which arm's signal is being detected.

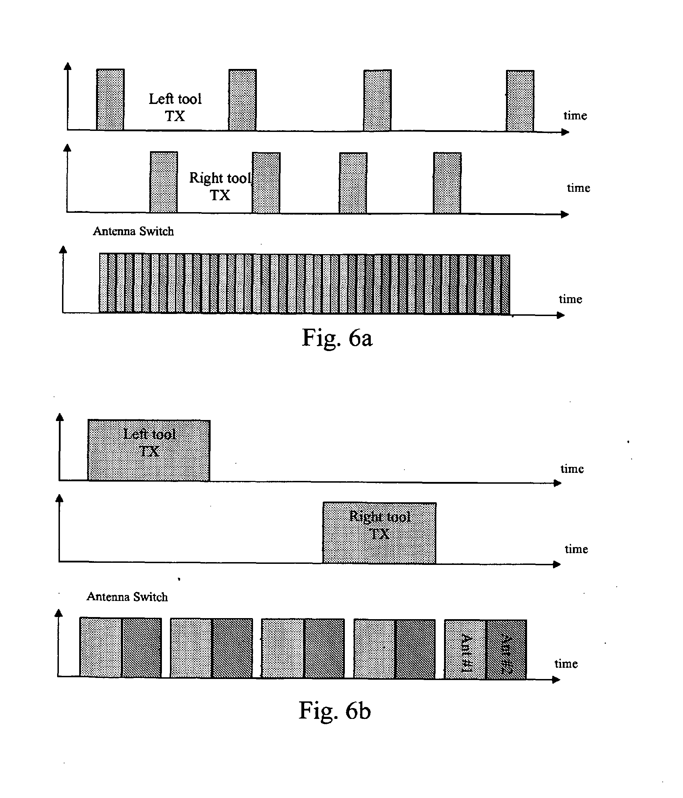

In order to ensure that the direction of a single transmission can be calculated (if only a single transmission is received), the "transmit on" duration is at least (N+1) X dwell intervals, where N is the number of antenna outputs. This ensures that the transmission is received during at least N successive dwell times, allowing the system to calculate its direction. For example if the receiver antenna is switched in sequence staying on for 10 ms (i.e., a 10 ms antenna dwell time) in each pattern out of two patterns, then the total antenna switch time cycle is 20 ms, and the transmitter switch on time is required to last for at least 30 msec. For example, in one embodiment of the invention, the transmit on/off cycle times are 120 ms and 150 ms for the left and right arm transmitter respectively. Each transmitter is on for 30 ms and off for the rest of the time. The antenna switch versus transmit periodic operation is shown in Fig. 6a.

In case of sequential transmission, each transmitter should be on for at least (N+1) X dwell intervals (receiver antenna dwell time). The antenna switch versus transmit sequential operation is shown in Fig. 6b.

In embodiments in which the two transmitters operate at different frequencies, the receiver scans all antenna patterns at the first frequency, then switches to the second frequency and scans all antenna patterns again, then returns to the first frequency, and so on.

Reference is now made to Fig. 7a, which illustrates an embodiment of the invention in which the directional antenna array has a planar structure. The short dipoles at each segment **72a,b,c** are covered to protect the wires and the circuits from humidity and mechanical fractures. The arms **73a,b,c** are made of any appropriate flexible material.

Reference is now made to Fig. 7b, which illustrates an embodiment of the invention in which the directional antenna array has a non-planar spatial structure. The fourth short dipole at segment **72d** is not located in the plane that contains segments **72a,b,c.** This arrangement allows the system to compute the spatial direction of the RF transmitter.

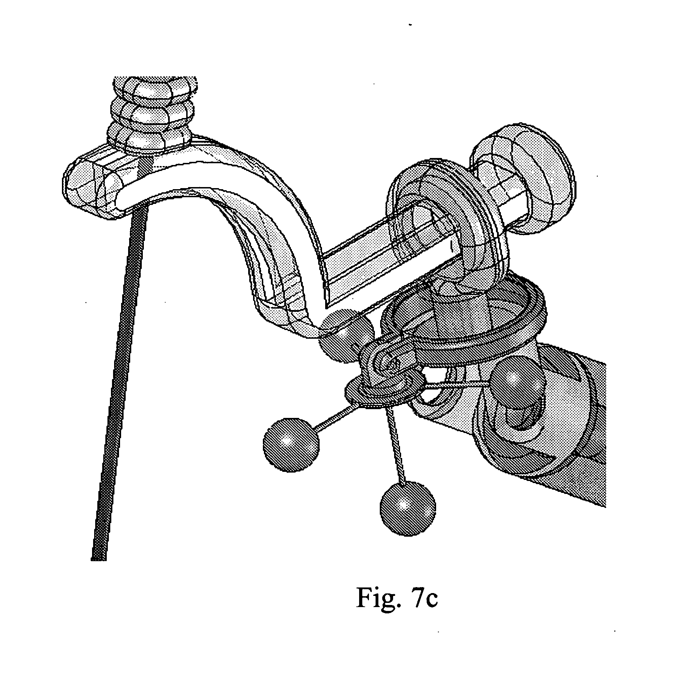

Reference is now made to Fig. 7c, which shows the antenna located on an automated automated assistant maneuvering system according to one embodiment of the invention.

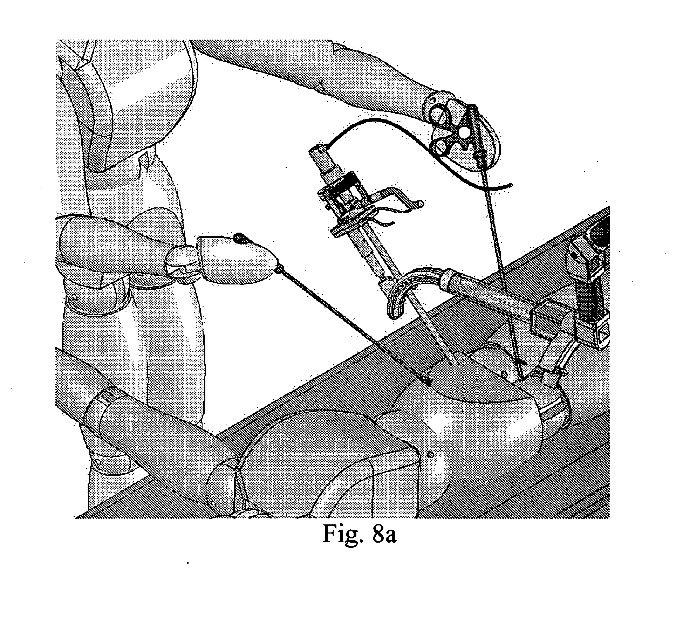

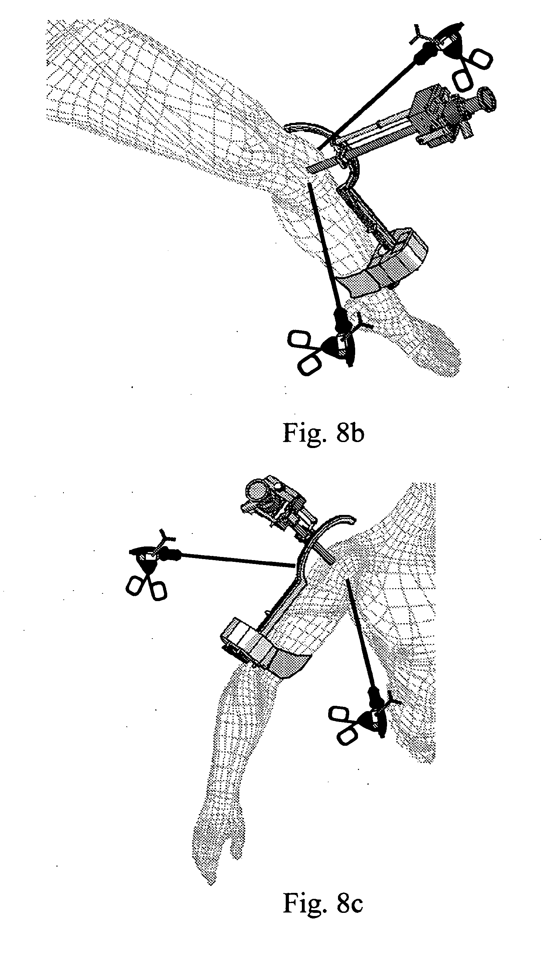

Reference is now made to Figs. 8a,b,c, which illustrate in a non-limiting manner some types of surgeries in which the location system disclosed in the present invention can be utilized.

Fig 8a shows an example of using the location system in abdominal laparoscopic surgery.

Fig 8b shows an example of using the location system in knee endoscopic surgery.

Lastly, Fig 8c shows an example of using the location system in shoulder endoscopic surgery.

## Claims

**1.** An interface between a surgeon and an automated assistant, comprising:

    a. at least one array comprising $N$ RF transmitters (11, 21), where $N$ is a positive integer;

    b. one RF receiver (31) provided with at least two co-located directional antenna; each of said co-located directional antenna is **characterized by** an antenna Gain Gr;

    c. means for attaching said RF transmitters (11, 21) to at least one surgical tool (10); and,

    d. a computerized operating system (40) adapted to record the received signal strength RSS received by each of said directional antenna of said one RF receiver and to calculate therefrom the position of each of said $N$ RF transmitters, and further adapted to provide automatically the results of said calculation to said surgeon; and **characterized in that**

said computerized operating system is adapted to calculate the angle from which the signal had been received by comparing said received signal strength received from all of said directional antenna of said one RF receiver (31) and the antenna's gain of each of said directional antenna of said one RF receiver (31) according to the following equation:

$$\text{RSS(directional antenna}_i) \ / \ \text{RSS(directional antenna}_k) \ = \ Gr\,(\text{directional antenna}_i) \ / \ Gr(\text{directional antenna}_k),$$

without the use of triangulation calculation and wherein the ratio of RSS(directional antenna$_i$) / RSS(directional antenna$_k$) is independent from the signal's transmit power and the distance between the RF transmitters (11, 21) and the one RF receiver (31), and wherein the ratio Gr(directional antenna$_i$) / Gr(directional antenna$_k$) is further defined by a known dependency of the angle from which said signal has been transmitted.

**2.** The interface of claim **1,** further comprising an endoscopic device; wherein said endoscopic device comprises optical imaging means, and further wherein said computerized operating system calculates at least one of the parameters chosen from the group consisting of (a) the spatial location of said at least one surgical tool (10); (b) the path of said at least one surgical tool (10); (c) the spatial location of the point of insertion of said at least one surgical tool (10) into the body of a patient; (d) the spatial location of the tip of said at least one surgical tool (10); (e) matching each RF transmitter code with each calculated spatial location of said at least one surgical tool (10) and/or said tip of said at least one surgical tool (10); (f) the predicted appearance of said at least one surgical tool (10) within said optical image; (g) if more than one of said at least one surgical tools appears simultaneously in said optical image, distinguishing among said more than at least surgical tools appearing in said optical image, and further wherein said computerized operating system provides automatically the results of said calculation to the human operator of said interface.

3. The interface of claim **1,** further comprising

   a. an endoscopic device;
   b. an automated assistant for said endoscopic device; and
   c. means for interfacing said computerized operating system to said automated assistant;

   wherein said computerized operating system calculates at least one of the parameters chosen from the group consisting of (a) the spatial location of said at least one surgical tool (10); (b) the path of said at least one surgical tool (10); (c) the spatial location of the point of insertion of said at least one surgical tool (10) into the body of a patient; (d) the spatial location of the tip of said at least one surgical tool (10); (e) matching each RF transmitter code with each calculated spatial location of said at least one surgical tool (10) and/or said tip of said at least one surgical tool (10); (f) a desired new location for said endoscopic device; (g) command protocol means for directing said automated assistant via said interface to maneuver said endoscopic device to a desired new location, and further wherein said computerized operating system provides automatically the results of said calculation to the human operator of said interface.

4. The interface of claim **3,** wherein said endoscopic device comprises optical imaging means, and further wherein said computerized operating system calculates at least one of the parameters chosen from the group consisting of (a) the spatial location of said at least one surgical tool (10); (b) the path of said at least one surgical tool (10); (c) the spatial location of the point of insertion of said at least one surgical tool (10) into the body of a patient; (d) the spatial location of the tip of said at least one surgical tool (10); (e) matching each RF transmitter code with each calculated spatial location of said at least one surgical tool and/or said tip of said at least one surgical tool (10); (f) the predicted appearance of said at least one surgical tool (10) within said optical image; (g) if more than one of said at least one surgical tools (10) appears simultaneously in said optical image, distinguishing among said more than at least surgical tools appearing in said optical image; (h) a desired new location for said optical imaging means; (i) a command protocol for directing said automated assistant via said interface to maneuver said endoscopic device to a desired new location, and further wherein said computerized operating system provides automatically the results of said calculation to the human operator of said interface.

5. The interface of claim **1,** wherein said interface is adapted for automatic operation, whereby each of said $N$ transmitters (11,21) transmits continuously.

6. The interface of claim **1,** wherein said interface is adapted for automatic operation, whereby each of said $N$ transmitters (11, 21) transmits continuously, and further wherein said computer transmits said calculated parameters for each of said $N$ transmitters(11, 21) in response to a command signal from the human operator of the interface.

7. The interface of claim **1,** wherein said receiver array is adapted to determine the angle whose vertex is the location of said antenna array and which is subtended by the line connecting any two of said $N$ transmitters.

8. The interface of claim **1,** wherein said $N$ RF transmitters transmit in the 430 MHz ISM band; further wherein said transmitters transmit a modulated signal, said modulation chosen from the group consisting of (a) frequency modulation occurs around a frequency of about *1.5* kHz, (b) amplitude modulation.

9. The interface of claim **8,** wherein each of said $N$ RF transmitters (11, 21) is modulated at a different frequency; further wherein said $N$ modulated frequencies are spanning range of frequencies from about 1.0 kHz to about 1.5 kHz.

10. The interface of claim **1**, wherein said receiver is a single conversion receiver.

11. A method for providing the 3D positional parameters of a surgical tool, comprising the steps of

   a. obtaining an interface, comprising (*i*) at least one array comprising $N$ RF transmitters (11, 21), where $N$ is a positive integer, (*ii*) one RF receiver (31) provided with at least two co-located directional antenna; each of said co-located directional antenna is **characterized by** an antenna Gain, Gr; (*iii*) a computerized operating system (40) adapted to record the received signal strength RSS received by each of said directional antenna of said RF receiver and further adapted to provide automatically the results of said calculation to the human operator of said interface;

b. obtaining a surgical tool (10);

c. attaching said RF transmitters (11, 21) to said surgical tool;

d. measuring the received signal strength RSS from said *N* RF transmitters (11, 21) received at each of said directional antenna of said RF receiver (31);

e. providing said 3D positional parameters relating to each of said *N* transmitters according to a predetermined protocol;

wherein said step of providing said 3D positional parameters of each of said *N* transmitters (11, 21) according to a predetermined protocol yields positional parameters of said surgical tool and is **characterized in that** said positional parameters comprise the angle from which the signal had been received, the angle from which the signal had been received being calculated by comparing said received signal strength received from all of said directional antenna in said RF receiver (31) and the antenna's gain of each of said directional antenna according to the following equation:

$$\text{RSS(directional antenna}_i) \text{ / RSS(directional antenna}_k) = \text{Gr(directional antenna}_i) \text{ / Gr(directional antenna}_k),$$

and

wherein the ratio Gr(directional antenna$_i$) / Gr(directional antenna$_k$) is **characterized by** a known dependency of the angle from which said signal has been transmitted, and wherein the ratio of RSS(directional antenna$_i$) / RSS(directional antenna$_k$) is independent from the signal's transmit power and the distance between the RF transmitters (11, 21) and the one RF receiver (31).

**12.** The method of claim **11**, additionally comprising step of transmitting either a continuously or a non-continuously signal from each of said N transmitters in response to a signal from the human operator of said interface.

**13.** The interface of claim **1**, wherein said computerized operating system is further adapted to calculate at least one of the parameters chosen from the group consisting of (a) the spatial location of said at least one surgical tool; (b) the path of said at least one surgical tool; (c) the spatial location of the point of insertion of said at least one surgical tool into the body of a patient; (d) the spatial location of the tip of said at least one surgical tool; (e) matching each RF transmitter code with each calculated spatial location of said at least one surgical tool and/or said tip of said at least one surgical tool; said computerized operating system performs said calculations and provides automatically the results of said calculations to the human operator of said interface.

**Patentansprüche**

**1.** Schnittstelle zwischen einem Chirurgen und einem automatisierten Assistenten, umfassend:

a. mindestens eine Anordnung, umfassend *N* HF-Sender (11, 21), wobei *N* eine positive ganze Zahl ist;

b. einen mit mindestens zwei co-lokalisierten Richtantennen versehenen HF-Empfänger (31); wobei jede der co-lokalisierten Richtantennen durch einen Antennengewinn Gr gekennzeichnet ist;

c. Mittel zum Befestigen der HF-Sender (11, 21) an mindestens einem chirurgischen Instrument (10); und

d. ein computergestütztes Betriebssystem (40), das zur Aufzeichnung der von jeder der Richtantennen des besagten einen HF-Empfängers empfangenen Empfangssignalstärke RSS und zum daraus Berechnen der Position jedes der besagten *N* HF-Sender eingerichtet ist, und weiter dazu eingerichtet ist, automatisch die Ergebnisse dieser Berechnung dem Chirurgen zur Verfügung zu stellen; und

**dadurch gekennzeichnet, dass**

das computergestützte Betriebssystem dazu eingerichtet ist, den Winkel, aus dem das Signal empfangen worden ist, durch Vergleichen der von allen der besagten Richtantennen des einen HF-Empfängers (31) empfangenen Empfangssignalstärke und dem Antennengewinn jeder der Richtantennen des einen HF-Empfängers (31) gemäß der folgenden Gleichung zu berechnen:

$$RSS(Richtantenne_i) / RSS(Richtantenne_k) = Gr(Richtantenne_i) / Gr(Richtantenne_k),$$

ohne die Anwendung von Triangulationsberechnung, und wobei das Verhältnis von $RSS(Richtantenne_i)$ / $RSS(Richtantenne_k)$ unabhängig von der Sendeleistung des Signals und dem Abstand zwischen den HF-Sendern (11, 21) und dem einen HF-Empfänger (31) ist, und wobei das Verhältnis $Gr(Richtantenne_i)$ / $Gr(Richtantenne_k)$ weiter durch eine bekannte Abhängigkeit von dem Winkel, aus dem das Signal übertragen wurde, definiert ist.

2. Schnittstelle nach Anspruch 1, weiter eine Endoskopievorrichtung umfassend; wobei die Endoskopievorrichtung optische Bildgebungsmittel umfasst, und weiter, wobei das computergestützte Betriebssystem mindestens einen der Parameter berechnet, der aus der Gruppe ausgewählt ist, bestehend aus (a) der Raumposition des mindestens einen chirurgischen Instruments (10); (b) dem Weg des mindestens einen chirurgischen Instruments (10); (c) der Raumposition des Eintrittspunktes des mindestens einen chirurgischen Instruments (10) in den Körper eines Patienten; (d) der Raumposition der Spitze des mindestens einen chirurgischen Instruments (10); (e) dem Abgleichen jedes HF-Sender-Codes mit jeder errechneten Raumposition des mindestens einen chirurgischen Instruments (10) und/oder der Spitze des mindestens einen chirurgischen Instruments (10); (f) dem vorhergesagten Erscheinen des mindestens einen chirurgischen Instruments (10) in der optischen Abbildung; (g) wenn mehr als eines des mindestens einen chirurgischen Instruments gleichzeitig in der optischen Abbildung erscheint, Unterscheiden zwischen den mehr als mindestens einem chirurgischen Instrumenten, die in der optischen Abbildung erscheinen, und weiter, wobei das computergestützte Betriebssystem die Ergebnisse dieser Berechnung automatisch der menschlichen Bedienperson der Schnittstelle vorlegt.

3. Schnittstelle nach Anspruch 1, weiter umfassend

    a. eine Endoskopievorrichtung;
    b. einen automatisierten Assistenten für diese Endoskopievorrichtung; und
    c. Mittel zur Verknüpfung des computergestützten Betriebssystems mit dem automatisierten Assistenten;

wobei das computergestützte Betriebssystem mindestens einen der Parameter berechnet, der aus der Gruppe ausgewählt ist, bestehend aus (a) der Raumposition des mindestens einen chirurgischen Instruments (10); (b) dem Weg des mindestens einen chirurgischen Instruments (10); (c) der Raumposition des Eintrittspunktes des mindestens einen chirurgischen Instruments (10) in den Körper eines Patienten; (d) der Raumposition der Spitze des mindestens einen chirurgischen Instruments (10); (e) dem Abgleichen jedes HF-Sender-Codes mit jeder errechneten Raumposition des mindestens einen chirurgischen Instruments (10) und/oder der Spitze des mindestens einen chirurgischen Instruments (10); (f) einer gewünschten neuen Position für die Endoskopievorrichtung; (g) Steuerungsprotokollmittel zum Lenken des automatisierten Assistenten mittels besagter Schnittstelle, um die Endoskopievorrichtung zu einer gewünschten neuen Position zu manövrieren, und weiter, wobei das computergestützte Betriebssystem die Ergebnisse dieser Berechnung automatisch der menschlichen Bedienperson der Schnittstelle vorlegt.

4. Schnittstelle nach Anspruch 3, wobei die Endoskopievorrichtung optische Bildgebungsmittel umfasst, und weiter, wobei das computergestützte Betriebssystem mindestens einen der Parameter berechnet, der aus der Gruppe ausgewählt ist, bestehend aus (a) der Raumposition des mindestens einen chirurgischen Instruments (10); (b) dem Weg des mindestens einen chirurgischen Instruments (10); (c) der Raumposition des Eintrittspunktes des mindestens einen chirurgischen Instruments (10) in den Körper eines Patienten; (d) der Raumposition der Spitze des mindestens einen chirurgischen Instruments (10); (e) dem Abgleichen jedes HF-Sender-Codes mit jeder errechneten Raumposition des mindestens einen chirurgischen Instruments und/oder der Spitze des mindestens einen chirurgischen Instruments (10); (f) dem vorhergesagten Erscheinen des mindestens einen chirurgischen Instruments (10) in der optischen Abbildung; (g) wenn mehr als eines der mindestens einen chirurgischen Instrumente (10) gleichzeitig in der optischen Abbildung erscheint, Unterscheiden zwischen den mehr als mindestens einem chirurgischen Instrumenten, die in der optischen Abbildung erscheinen; (h) einer gewünschten neuen Position für die optischen Bildgebungsmittel; (i) einem Steuerungsprotokoll zum Lenken des automatisierten Assistenten mittels besagter Schnittstelle, um die Endoskopievorrichtung zu einer gewünschten neuen Position zu manövrieren, und weiter, wobei das computergestützte Betriebssystem die Ergebnisse dieser Berechnung automatisch der menschlichen Bedienperson der Schnittstelle vorlegt.

5. Schnittstelle nach Anspruch 1, wobei die Schnittstelle zum automatischen Betrieb eingerichtet ist, wobei jeder der *N* HF-Sender (11, 21) kontinuierlich sendet.

6. Schnittstelle nach Anspruch 1, wobei die Schnittstelle zum automatischen Betrieb eingerichtet ist, wobei jeder der *N* Sender (11, 21) kontinuierlich sendet, und wobei weiter der Computer die errechneten Parameter für jeden der *N* Sender (11, 21) in Reaktion auf ein Befehlssignal von der menschlichen Bedienperson der Schnittstelle überträgt.

7. Schnittstelle nach Anspruch 1, wobei die Empfängeranordnung dazu eingerichtet ist, den Winkel zu bestimmen, dessen Scheitel der Standort der Antennenanordnung ist und dem die Linie gegenüberliegt, die gleich welche zwei der *N* Sender verbindet.

8. Schnittstelle nach Anspruch 1, wobei die *N* HF-Sender im 430 MHz ISM-Band senden; weiter, wobei die Sender ein moduliertes Signal übertragen, wobei die Modulation aus der Gruppe ausgewählt ist, bestehend aus (a) Frequenzmodulation, die um eine Frequenz von etwa 1,5 kHz stattfindet, (b) Amplitudenmodulation.

9. Schnittstelle nach Anspruch 8, wobei jeder der *N* HF-Sender (11, 21) auf einer unterschiedlichen Frequenz moduliert wird; wobei weiter die *N* modulierten Frequenzen den Frequenzbereich von etwa 1,0 kHz bis etwa 1,5 kHz überspannen.

10. Schnittstelle nach Anspruch 1, wobei der Empfänger ein Einfachüberlagerungsempfänger ist.

11. Verfahren zur Bereitstellung der 3D-Positionsparameter eines chirurgischen Instruments, umfassend die Schritte des:

    a. Beziehens einer Schnittstelle, umfassend (*i*) mindestens eine Anordnung umfassend *N* HF-Sender (11, 21), wobei *N* eine positive ganze Zahl ist; (ii) einen mit mindestens zwei co-lokalisierten Richtantennen versehenen HF-Empfänger (31); wobei jede der co-lokalisierten Richtantennen durch einen Antennengewinn, Gr, gekennzeichnet ist; (iii) ein computergestütztes Betriebssystem (40), das zur Aufzeichnung der von jeder der Richtantennen des besagten HF-Empfängers empfangenen Empfangssignalstärke RSS eingerichtet ist und weiter dazu eingerichtet ist, automatisch die Ergebnisse dieser Berechnung der menschlichen Bedienperson dieser Schnittstelle vorzulegen;
    b. Beziehens eines chirurgischen Instruments (10);
    c. Befestigens der HF-Sender (11, 21) an dem chirurgischen Instrument;
    d. Messens der Empfangssignalstärke RSS von den *N* HF-Sendern (11, 21), die an jeder der Richtantennen des HF-Empfängers (31) empfangen wird;
    e. Bereitstellens dieser 3D-Positionsparameter, die sich auf jeden der *N* Sender beziehen, gemäß einem vorbestimmten Protokoll;

wobei der Schritt des Bereitstellens dieser 3D-Positionsparameter jedes der *N* Sender (11, 21) gemäß einem vorbestimmten Protokoll Positionsparameter des chirurgischen Instruments ergibt und **dadurch gekennzeichnet ist, dass** die Positionsparameter den Winkel umfassen, aus dem das Signal empfangen worden war, wobei der Winkel, aus dem das Signal empfangen worden war, berechnet wird durch Vergleichen der von allen Richtantennen in dem HF-Empfänger (31) empfangenen Empfangssignalstärke und dem Antennengewinn jeder der Richtantennen, gemäß der folgenden Gleichung:

$$\mathrm{RSS}(\mathrm{Richtantenne}_i) \;/\; \mathrm{RSS}(\mathrm{Richtantenne}_k) = \mathrm{Gr}(\mathrm{Richtantenne}_i) \;/\; \mathrm{Gr}(\mathrm{Richtantenne}_k),$$

und
wobei das Verhältnis $\mathrm{Gr}(\mathrm{Richtantenne}_i) / \mathrm{Gr}(\mathrm{Richtantenne}_k)$ durch eine bekannte Abhängigkeit von dem Winkel, aus dem das Signal übertragen wurde, gekennzeichnet ist und wobei das Verhältnis von $\mathrm{RSS}(\mathrm{Richtantenne}_i) / \mathrm{RSS}(\mathrm{Richtantenne}_k)$ unabhängig von der Sendeleistung des Signals und dem Abstand zwischen den HF-Sendern (11, 21) und dem einen HF-Empfänger (31) ist.

12. Verfahren nach Anspruch 11, zusätzlich den Schritt des Übertragens entweder eines kontinuierlichen oder eines nicht kontinuierlichen Signals von jedem der N Sender in Reaktion auf ein Signal von der menschlichen Bedienperson der Schnittstelle umfassend.

13. Schnittstelle nach Anspruch 1, wobei das computergestützte Betriebssystem weiter dazu eingerichtet ist, mindestens einen der Parameter zu berechnen, der aus der Gruppe ausgewählt ist, bestehend aus (a) der Raumposition des mindestens einen chirurgischen Instruments; (b) dem Weg des mindestens einen chirurgischen Instruments; (c) der Raumposition des Eintrittspunktes des mindestens einen chirurgischen Instruments in den Körper eines Patienten; (d) der Raumposition der Spitze des mindestens einen chirurgischen Instruments; (e) dem Abgleichen jedes HF-Sender-Codes mit jeder errechneten Raumposition des mindestens einen chirurgischen Instruments und/oder der Spitze des mindestens einen chirurgischen Instruments; wobei das computergestützte Betriebssystem diese Berechnungen durchführt und die Ergebnisse dieser Berechnung automatisch der menschlichen Bedienperson der Schnittstelle vorlegt.

**Revendications**

1. Interface entre un chirurgien et un assistant automatisé, comprenant:

    a. au moins un réseau comprenant N émetteurs-récepteurs (11, 21), N représentant un entier positif ;
    b. un récepteur RF (31) muni d'au moins deux antennes directionnelles adjacentes, chacune desdites antennes directionnelles adjacentes étant **caractérisée par** un gain d'antenne Gr;
    c. un moyen pour fixer lesdits émetteurs-récepteurs RF (11, 21) à au moins un instrument chirurgical (10) ; et
    d. un système opératoire informatisé (40) conçu pour enregistrer la force du signal reçu RSS reçu par chacune desdites antennes directionnelles dudit un récepteur RF et pour calculer, à partir de là, la position de chacun desdits *N* émetteurs-récepteurs RF, et conçu en outre pour fournir automatiquement les résultats dudit calcul audit chirurgien ; et **caractérisée en ce que**

    ledit système opératoire informatisé est conçu pour calculer l'ongle à partir duquel le signal a été reçu en comparant ladite force du signal reçu à partir de l'ensemble desdites antennes directionnelles dudit un récepteur RF (31) et le gain d'antenne de chacune desdites antennes directionnelles dudit un récepteur RF (31) conformément à l'équation suivante : RSS (antenne directionnelle$_i$) / RSS (antenne directionnelle$_k$) = Gr (antenne directionnelle$_i$) / Gr (antenne directionnelle$_k$), sans l'utilisation d'un calcul de triangulation et, dans laquelle le rapport RSS (antenne directionnelle$_i$) / RSS (antenne directionnelle$_k$) est indépendant de la puissance de transmission du signal et de la distance entre les émetteurs-récepteurs RF (11, 21) et ledit un récepteur RF (31), et dans laquelle le rapport Gr (antenne directionnelle$_i$) / Gr (antenne directionnelle$_k$) est en outre défini par une dépendance connue de l'ongle à partir duquel ledit signal a été transmis.

2. Interface selon la revendication 1, comprenant en outre un dispositif endoscopique, ledit dispositif endoscopique comprenant un moyen d'imagerie optique, et en outre dans laquelle ledit système opératoire informatisé calcule au moins un des paramètres choisis parmi le groupe constitué par : (a) l'emplacement dans l'espace dudit au moins un instrument chirurgical (10) ; (b) le chemin dudit au moins un instrument chirurgical (10) ; (c) l'emplacement dans l'espace du point d'insertion dudit au moins un instrument chirurgical (10) dans le corps d'un patient; (d) l'emplacement dans l'espace de l'extrémité dudit au moins un instrument chirurgical (10) ; (e) la mise en correspondance du code de chaque émetteur-récepteur RF avec chaque emplacement calculé dans l'espace dudit au moins un instrument chirurgical (10) et/ou de ladite extrémité dudit au moins un instrument chirurgical (10) ; (f) l'aspect prévu dudit au moins un instrument chirurgical (10) au sein de ladite image optique ; (g) lorsque plus d'un dudit au moins un instrument chirurgical apparaît simultanément dans ladite image optique, opérer une distinction parmi lesdits plus d'un dudit au moins un instrument chirurgical apparaissant dans ladite image optique ; et en outre dans laquelle ledit système opératoire informatisé fournit automatiquement les résultats dudit calcul à l'opérateur humain de ladite interface.

3. Interface selon la revendication 1, comprenant en outre :

    a. un dispositif endoscopique ;
    b. un assistant automatisé pour ledit dispositif endoscopique ; et
    c. un moyen pour la liaison par interface dudit système opératoire informatisé audit assistant automatisé ;

    dans laquelle ledit système opératoire informatisé calcule au moins un des paramètres choisis parmi le groupe constitué par: (a) l'emplacement dans l'espace dudit au moins un instrument chirurgical (10) ; (b) le chemin dudit au moins un instrument chirurgical (10) ; (c) l'emplacement dans l'espace du point d'insertion dudit au moins un instrument chirurgical (10) dans le corps d'un patient; (d) l'emplacement dans l'espace de l'extrémité dudit au moins un instrument chirurgical (10) ; (e) la mise en correspondance du code de chaque émetteur-récepteur RF avec chaque emplacement calculé dans l'espace dudit au moins un instrument chirurgical (10) et/ou de ladite extrémité dudit au moins un instrument chirurgical (10) ; (f) un nouvel emplacement souhaité pour ledit dispositif endoscopique ; (g) un moyen de protocole de commande pour diriger ledit assistant automatisé via ladite interface pour aiguiller ledit dispositif endoscopique vers un nouvel emplacement désiré ; et en outre dans laquelle ledit système opératoire informatisé fournit automatiquement les résultats dudit calcul à l'opérateur humain de ladite interface.

4. Interface selon la revendication 3, dans laquelle ledit dispositif endoscopique comprend un moyen d'imagerie optique, et en outre dans laquelle ledit système opératoire informatisé calcule au moins un des paramètres choisis parmi le groupe constitué par : (a) l'emplacement dans l'espace dudit au moins un instrument chirurgical (10) ; (b) le chemin dudit au moins un instrument chirurgical (10) ; (c) l'emplacement dans l'espace du point d'insertion dudit au moins un instrument chirurgical (10) dans le corps d'un patient ; (d) l'emplacement dans l'espace de l'extrémité dudit au moins un instrument chirurgical (10) ; (e) la mise en correspondance du code de chaque émetteur-récepteur RF avec chaque emplacement calculé dans l'espace dudit au moins un instrument chirurgical (10) et/ou de ladite extrémité dudit au moins un instrument chirurgical (10) ; (f) l'aspect prévu dudit au moins un instrument chirurgical (10) au sein de ladite image optique ; (g) lorsque plus d'un dudit au moins un instrument chirurgical apparaît simultanément dans ladite image optique, opérer une distinction parmi lesdits plus d'un dudit au moins un instrument chirurgical apparaissant dans ladite image optique ; (h) un nouvel emplacement souhaité pour ledit dispositif endoscopique ; (i) un moyen de protocole de commande pour diriger ledit assistant automatisé via ladite interface pour aiguiller ledit dispositif endoscopique vers un nouvel emplacement désiré ; et en outre dans laquelle ledit système opératoire informatisé fournit automatiquement les résultats dudit calcul à l'opérateur humain de ladite interface.

5. Interface selon la revendication 1, dans laquelle ladite interface est conçue pour une opération automatique, chacun desdits *N* émetteurs-récepteurs (11, 21) faisant l'objet d'une transmission en continu.

6. Interface selon la revendication 1, dans laquelle ladite interface est conçue pour une opération automatique, chacun desdits *N* émetteurs-récepteurs (11, 21) faisant l'objet d'une transmission en continu, et en outre dans laquelle ledit ordinateur transmet lesdits paramètres calculés pour chacun desdits N émetteurs-récepteurs (11, 21) en réponse à un signal de commande émis par l'opérateur humain de l'interface.

7. Interface selon la revendication 1, dans laquelle ledit réseau de récepteurs est conçu pour déterminer l'ongle dont le sommet représente l'emplacement dudit réseau d'antennes et qui est sous-tendu par la ligne reliant l'un quelconque de deux desdits N émetteurs-récepteurs.

8. Interface selon la revendication 1, dans laquelle lesdits *N* émetteurs-récepteurs émettent dans la bande industrielle, scientifique et médicale de 430 MHz; en outre dans laquelle lesdits émetteurs-récepteurs transmettent un signal modulé, ladite modulation étant choisie parmi le groupe constitué par (a) une modulation de fréquence qui a lieu autour d'une fréquence d'environ 1,5 kHz, (b) une modulation d'amplitude.

9. Interface selon la revendication 8, dans laquelle chacun desdits *N* émetteurs-récepteurs (11, 21) est modulé à une fréquence différente ; en outre dans laquelle lesdites *N* fréquences modulées s'étendent sur une plage de fréquences d'environ 1,0 kHz à environ 1,5 kHz.

10. Interface selon la revendication 1, dans laquelle ledit récepteur est un récepteur à un seul changement de fréquence.

11. Procédé pour procurer les paramètres de position en trois dimensions d'un instrument chirurgical, comprenant les étapes consistant à :

a. obtenir une interface comprenant (*i*) au moins un réseau comprenant *N* émetteurs-récepteurs (11, 21), *N* représentant un entier positif ; (*ii*) un récepteur RF (31) muni d'au moins deux antennes directionnelles adjacentes, chacune desdites antennes directionnelles adjacentes étant **caractérisée par** un gain d'antenne Gr ; (*iii*) un système opératoire informatisé (40) conçu pour enregistrer la force du signal reçu RSS reçu par chacune desdites antennes directionnelles dudit un récepteur RF, et conçu en outre pour fournir automatiquement les résultats dudit calcul à l'opérateur humain de ladite interface ;

b. obtenir un instrument chirurgical (10) ;

c. fixer lesdits émetteurs-récepteurs RF (11, 21) audit instrument chirurgical ;

d. mesurer la force du signal reçu RSS à partir desdits *N* émetteurs-récepteurs (11, 21), reçu à chacune desdites antennes directionnelles dudit récepteur RF (31) ;

e. procurer lesdits paramètres de position en trois dimensions par rapport à chacun desdits *N* émetteurs-récepteurs conformément à un protocole prédéterminé ;

dans lequel ladite étape consistant à procurer lesdits paramètres de position en trois dimensions de chacun desdits *N* émetteurs-récepteurs (11, 21) conformément à un protocole prédéterminé permet d'obtenir les paramètres de position dudit instrument chirurgical, et est **caractérisée en ce que** lesdits paramètres de position comprennent l'ongle à partir duquel le signal a été reçu, l'ongle à partir duquel le signal a été reçu étant calculé en comparant ladite force de signal reçu, reçu à partir de l'ensemble desdites antennes directionnelles dans ledit récepteur RF (31), et le gain d'antenne de chacune desdites antennes directionnelles conformément à l'équation suivante : RSS (antenne directionnelle$_i$) / RSS (antenne directionnelle$_k$) = Gr (antenne directionnelle$_i$) / Gr (antenne directionnelle$_k$), le rapport Gr (antenne directionnelle$_i$) / Gr (antenne directionnelle$_k$) étant **caractérisé par** une dépendance connue de l'ongle à partir duquel ledit signal a été transmis, et le rapport RSS (antenne directionnelle$_i$) / RSS (antenne directionnelle$_k$) étant indépendant de la puissance de transmission du signal et de la distance entre les émetteurs-récepteurs RF (11, 21) et ledit un récepteur RF (31).

**12.** Procédé selon la revendication 11, comprenant en outre l'étape consistant à transmettre, soit un signal continu, soit un signal non continu à partir de chacun desdits *N* émetteurs-récepteurs en réponse à un signal fourni par l'opérateur humain de ladite interface.

**13.** Interface selon la revendication 1, dans laquelle ledit système opératoire informatisé est en outre conçu pour calculer au moins un des paramètres choisis parmi le groupe constitué par: (a) l'emplacement dans l'espace dudit au moins un instrument chirurgical ; (b) le chemin dudit au moins un instrument chirurgical ; (c) l'emplacement dans l'espace du point d'insertion dudit au moins un instrument chirurgical dans le corps d'un patient; (d) l'emplacement dans l'espace de l'extrémité dudit au moins un instrument chirurgical ; (e) la mise en correspondance du code de chaque émetteur-récepteur RF avec chaque emplacement calculé dans l'espace dudit au moins un instrument chirurgical et/ou de ladite extrémité dudit au moins un instrument chirurgical ; ledit système opératoire informatisé réalisant lesdits calculs et procurant de manière automatique les résultats desdits calculs à l'opérateur humain de ladite interface.

Fig1a.

Fig. 1b

Fig 2a

Fig 2b

Fig 2c

Antenna pattern for left and right

dirrection in deg

Fig. 3a

$Gr(antenna_j) -$
$Gr(antenna_k)$

Angle

Fig. 3b

Fig. 4a

Fig. 4b

RF in

BPF

Variable
Attenuator

Quadrate Mixer

I channel baseband

LPF

Power
detect

X

LPF

Power
detect

Q channel baseband

Switch

Local
Oscillator

Reference
XTAL osc

PLL

ADC

Frequency control

Receiver I/Q
power output

AGC attenuation

## Fig. 4c

ADC signal

Input/
output
interface

Control
μ-processor

Report Left / Right

Frequency
control

AGC attenuation

RAM /Flash

Antenna switch
control

## Fig. 4d

Receiver
ADC out

Average
amplitude

X

X >
threshold

Save
Aver_A(n)
= X

Calculate
signal
direction

AM
demodulator

Estimate
modulating
frequency

Arm = Left
/right / cant
decide

Fig. 5

Fig. 6a

Fig. 6b

Fig. 7a

Fig. 7b

Fig. 7c

Fig. 8a

Fig. 8b

Fig. 8c

**EP 2 208 463 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6714841 B
- US 2003073935 A
- US 5443066 A

**Non-patent literature cited in the description**

- **K. ARSHAK.** A method for estimating the real time Positions of a moving object in wireless telemetry applications using RF sensors. *IEEE Sensors Applications Symposium, San Diego, California, USA,* 06 February 2007